# EUROPÄISCHE PATENTANMELDUNG

(11) **EP 0 726 284 A1**
(43) Veröffentlichungstag der Anmeldung: **14.08.1996**
(21) Anmeldenummer: 95120215.9
(22) Anmeldetag: 20.12.1995
(51) Int. Cl.: C08G 18/80, C07C 271/06

(54) **Blockierte Polyisocyanate, Verfahren zu ihrer Herstellung und daraus hergestellte Lacke und Beschichtungssysteme**

(30) Priorität: 11.02.1995 DE 19504530
(71) Anmelder: HÜLS AKTIENGESELLSCHAFT, D-45764 Marl (DE)
(72) Erfinder: Andrejewski, Werner, D-46284 Dorsten (DE); Lomölder, Rainer, Dr., D-48153 Münster (DE); Wenning, Andreas, Dr., D-48301 Nottuln (DE)

(57) **Zusammenfassung**

Blockierte Polyisocyanate mit einem freien NCO-Gehalt von kleiner 2 Massen-%, dadurch gekennzeichnet, daß sie mit α-Hydroxycarbonsäureestern der allgemeinen Formel I 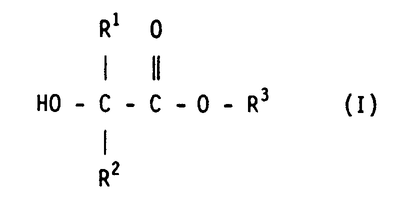 blockiert sind, wobei R¹ und R² gleichzeitig oder unabhängig voneinander Wasserstoff oder Alkyl-, Alkenyl-, Aralkyl- oder Arylgruppen bedeuten und R³ eine Alkyl-, Alkenyl-, Aralkyl- oder Arylgruppe darstellt und wobei diese weitere, auch Heteroatome enthaltende Substituenten aufweisen können.

## Beschreibung

Die vorliegende Erfindung betrifft blockierte Polyisocyanate, ein Verfahren zu ihrer Herstellung und ihre Verwendung in Klebstoffen und Lacken, insbesondere in 1K-PUR-Einbrennlacken sowie anderen Beschichtungssystemen.

Die Blockierung von Polyisocyanaten zum zeltweisen Schutz der NCO-Gruppen ist eine seit langem bekannte Arbeitsmethode und wird z. B. im Houben-Weyl, Methoden der organischen Chemie XIV/2, S. 61-70, beschrieben. Härtbare Zusammensetzungen, die blockierte Polyisocyanate enthalten, finden z. B. Verwendung in Polyurethan-Lacken. Die Härtung der Lackfilme geschieht durch Reaktion der thermisch reaktivierten Polyisocyanate mit Polyolen. Eine Zusammenstellung prinzipiell geeigneter Blockierungsmittel findet sich z. B. bei Zeno W. Wicks, Jr., Progress in Organic Coatings 3 (1975) 73-79, 9 (1981) 3-28.

Eine Vielzahl solcher Blockierungsmittel benötigt in Lackanwendungen zur Härtung bei einer Zeit von 20 bis 30 Minuten Temperaturen von über 160 °C. Aus ökonomischer Sicht weisen sie damit zu hohe Deblockierungstemperaturen auf.

In jüngster Vergangenheit sind einige wenige neue Systeme mit niedrigen Einbrenntemperaturen um 100 bis 140 °C und Einbrennzeiten von 20 bis 30 Minuten gefunden worden. So werden Oxim-blockierte Polyisocyanate z. B. in der DE-OS 22 00 342, EP-A-0 432 257, USP 3 857 818 beschrieben. In USP 4 452 681, USP 4 452 930 und USP 4 452 963 werden Urethangruppen-enthaltende Verbindungen, dargestellt aus Polyisocyanaten und α-Hydroxysäureamiden, beschrieben, die als Niedrigtemperatur-Vernetzer von Lösemittelbasierender als auch im besonderen Wasser-basierender Zusammensetzungen, wie Elektrotauchlacken, Verwendung finden. In der USP 4 976 837 sowie in der EP-A-0 500 495 werden Pyrazole zur Herstellung von Einbrennlacken beansprucht. Die Oxim- bzw. α-Hydroxysäureamid-blockierten Polyisocyanate neigen aufgrund der Abspaltung von aminhaltigen Verbindungen, die teilweise in der Beschichtung verbleiben, zur Vergilbung. Die Eigenschaften der mit Pyrazol blockierten Polyisocyanate bei der Herstellung von Einbrennlacken bezüglich Reaktivität und Härtungszeiten sind sehr stark abhängig vom verwendeten Blockierungsmittel/Polyisocyanat-System. Nur in wenigen Systemen sind Vorteile erkennbar.

Der Erfindung lag nun die Aufgabe zugrunde, neue, kostengünstige blockierte Polyisocyanate zur Verfügung zu stellen, die die Blockierungsmittel bei niedrigeren Temperaturen abspalten, aber dennoch bei Umgebungstemperaturen stabil bleiben. Solche um 120 bis 130 °C deblockierenden Polyisocyanate sollten sich insbesondere auch in Kombination mit geeigneten Polyolkomponenten zur Herstellung von 1K-Einbrennlacken eignen, die eine geringe Vergilbungsneigung aufweisen.

Überraschenderweise konnte diese Aufgabe dadurch gelöst werden, daß zur Blockierung von Polyisocyanaten Alkohole mit benachbarten Esterfunktionen allein oder in Verbindung mit den bekannten Blockierungsmitteln eingesetzt werden.

Gegenstand der vorliegenden Erfindung sind somit blockierte Polyisocyanate mit einem freien NCO-Gehalt von kleiner 2 Massen-%, dadurch gekennzeichnet, daß sie mit α-Hydroxycarbonsäureestern der allgemeinen Formel I 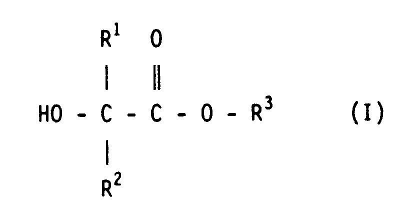 blockiert sind, wobei R¹ und R² gleichzeitig oder unabhängig voneinander Wasserstoff oder Alkyl-, Alkenyl-, Aralkyl- oder Arylgruppen bedeuten und R³ eine Alkyl-, Alkenyl-, Aralkyl- oder Arylgruppe darstellt und wobei diese weitere, auch Heteroatome enthaltende Substituenten aufweisen können.

Bevorzugte α-Hydroxycarbonsäureester sind solche, in denen R¹ Wasserstoff, R² Wasserstoff oder eine Methylgruppe und R³ ein Alkylrest mit 1 bis 10 Kohlenstoffatomen darstellen.

Diese erfindungsgemäßen Verbindungen zeichnen sich durch einen blockierten NCO-Gehalt von 5 bis 25 Massen-% und einem freien NCO-Gehalt von kleiner 2.0 Massen-% aus. Ihre Konsistenz bei Umgebungstemperatur ist flüssig oder fest.

Die erfindungsgemäßen blockierten Polyisocyanate können so hergestellt werden, indem man Polyisocyanate mit α-Hydroxycarbonsäureestern der allgemeinen Formel I 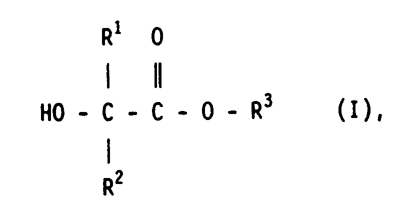 wobei R¹ und R² gleichzeitig oder unabhängig voneinander Wasserstoff oder Alkyl-, Alkenyl-, Aralkyl- oder Arylgruppen bedeuten und R³ eine Alkyl-, Alkenyl-, Aralkyl- oder Arylgruppen darstellt und wobei diese weitere, auch Heteroatome enthaltende Substituenten aufweisen können, so umsetzt, daß pro 0,8 bis 1,2 mol Blockierungsmittel 1 NCO-Äquivalent der Polyisocyanatkomponente in Gegenwart von 0,01 bis 2,0 Massen-% eines Katalysators zur Reaktion kommen.

Das zu blockierende Polyisocyanat kann jedes organische Polyisocyanat sein, welches zur Vernetzung von Verbindungen mit aktives Wasserstoff geeignet ist, d. h. aliphatische einschließlich cycloaliphatische, aromatische und heterocyclische Polyisocyanate mit mindestens zwei Isocyanatgruppen und Gemische hiervon. Repräsentative Beispiele der Polyisocyanate sind aliphatische Isocyanate wie Alkylenisocyanate, z. B. Ethylendiisocyanat, Propylendiisocyanat, Tetramethylendiisocyanat, Pentamethylendiisocyanat, 3-Methylpentamethylen-1,5-diisocyanat (MPDI), Hexamethylendiisocyanat (HDI), Trimethylhexamethylen-1,6-diisocyanat (TMDI), insbesondere das 2,2,4- und das 2,4,4-Isomere und technische Gemische beider Isomere, Decamethylendiisocyanat und Dodecamethylendiisocyanat sowie Cycloalkylenisocyanate, z. B. 1,3-Cyclopentyldiisocyanat, 1,2-Cyclohexyldiisocyanat, 1,4-Cyclohexyldiisocyanat, ω,ω'-Diisocyanato-1,4-dimethylcyclohexan, ω,ω'-Diisocyanato-1,3-dimethylcyclohexan, 1-Methyl-2,4-diisocyanatocyclohexan, 4,4'-Methylen-bis(cyclohexylisocyanat) (H₁₂MDI), und 3,5,5-Trimethyl-1-isocyanato-3-isocyanatomethylcyclohexan (IPDI). Repräsentative Beispiele für Polyisocyanate sind aromatische Isocyanate wie Arylisocyanate, z. B. m-Phenylendiisocyanat, p-Phenylendiisocyanat, 4,4'-Diphenyldiisocyanat, 1,4-Naphthalendiisocyanat und 1,5-Naphthalendiisocyanat sowie Alkarylisocyanate, z. B. Diisocyanato-diphenylmethan (MDI), insbesondere das 4,4'-Isomere, aber auch technische Gemische verschiedener Isomere, beispielsweise der 4,4'- und 2,4'-Isomere, Diisocyanatomethyl-benzol (TDI), insbesondere das 2,4- und das 2,6- Isomere und technische Gemische beider Isomere, 4,4'-Toluidindiisocyanat, 1,3-Bis(isocyanato-methyl)benzol (XDI), m-Tetramethylxylylen-diisocyanat (TMXDI) und Polymethylenpolyphenylisocyanat, kernsubstituierte aromatische Isocyanate, z. B. Dianisidindiisocyanat, 4,4'-Diphenyletherdiisocyanat und Chlordiphenyldiisocyanat. Ebenfalls gut geeignet sind auch Polyisocyanate, die durch Umsetzung von Polyisocyanaten mit sich selbst über Isocyanatgruppen erhältlich sind, wie Uretdione oder Carbodiimidverbindungen, die durch Reaktion zweier Isocyanatgruppen entstehen, oder wie Isocyanurate, die durch Reaktion dreier Isocyanatgruppen gebildet werden. Die Polyisocyanate können ebenfalls Biuret- oder Allophanatgruppen enthalten. Für die Erfindung gut geeignet sind auch Polyisocyanatpräpolymere, die durchschnittlich mehr als eine Isocyanatgruppe pro Molekül aufweisen. Sie werden durch Vorreaktion eines molaren Überschusses beispielsweise eines der obengenannten Polyisocyanate mit einem organischen Material erhalten, das mindestens zwei aktive Wasserstoffatome pro Molekül aufweist, z. B. in Form von Hydroxylgruppen wie bei Polyalkylenpolyolen.

Repräsentative Beispiele der Blockierungsmittel sind Glykolsäuremethylester, Glykolsäureethylester, Glykolsäurebutylester, Milchsäureethylester und Milchsäurebutylester.

Die Herstellung der erfindungsgemäßen blockierten Polyisocyanate erfolgt durch Umsetzung von 0,8 bis 1,2 mol des Blockierungsmittels mit 1 NCO-Äquivalent des Polyisocyanates. Gegebenenfalls können 0,01 bis 2,0 Massen-%, vorzugsweise 0,05 bis 1,0 Massen-%, eines Katalysators zugesetzt werden. Als Katalysatoren werden konventionelle Urethanisierungskatalysatoren eingesetzt. Derartige Katalysatoren sind z. B. Metalle, Metallsalze oder Metallkomplexe wie Bleitetraacetat, Di- butylzinndilaurat, Zinkoktoat oder tertiäre Amine wie Triethylamin oder 1.4-Diazabicyclo[2.2.2]-octan. Die Umsetzung kann bei Raumtemperatur oder höheren Temperaturen, beispielsweise zwischen 50 und 130 °C durchgeführt werden.

Die Blockierung kann in Substanz oder aber in Gegenwart geeigneter Lösemittel erfolgen. Ob lösemittelfrei gearbeitet wird, hängt weitgehend vom späteren Einsatzgebiet des blockierten Polyisocyanates ab. Wird es zur Herstellung von hitzehärtbaren PUR-Pulvern eingesetzt, erfolgt seine Herstellung lösemittelfrei. Beim Einsatz der erfindungsgemäßen blockierten Polyisocyanate als Härterkomponente zur Herstellung lösemittelhaltiger 1K-PUR-Einbrennlacke bietet sich eine Darstellung in Lösung an. Geeignete inerte Lösemittel sind aromatische Kohlenwasserstoffe, Ester oder Ketone und als Verschnitt- oder Verdünnungsmittel aliphatische Kohlenwasserstoffe.

Die Blockierung des Polyisocyanates mit dem Blockierungsmittel findet in der Regel so statt, daß zu dem auf ca. 25 bis 60 °C erhitzten Polyisocyanat mit 0,05 bis 1,0 Massen-% Katalysator Dibutylzinndilaurat, bezogen auf die Summe aus Polyisocyanat und Blockierungsmittel, in ca. 20 bis 40 Massen-% inertem Lösungsmittel das Blockierungsmittel so zudosiert wird, daß die Temperatur des Reaktionsgemisches 100 °C nicht übersteigt. Nach Beendigung der Zugabe des Blockierungsmittels wird das Gemisch noch so lange auf Reaktionstemperatur gehalten, bis praktisch eine vollständige Umsetzung stattgefunden hat. In der Regel kommt 1 NCO-Äquivalent der Isocyanatkomponente mit 1 mol Blockierungsmittel zur Reaktion.

Im Rahmen der Erfindung ist es auch möglich, die erfindungsgemäß eingesetzten α-Hydroxycarbonsäureester teilweise durch bekannte Blockierungsmittel zu ersetzen. Demnach können die blockierten Polyisocyanate mindestens 10 Massen-%, bevorzugt mindestens 50 Massen-%, Reste von α-Hydroxycarbonsäureester und anderen Blockierungsmitteln, wie z. B. Oxime oder sekundäre Amine, enthalten, sofern die Kombination den gestellten Anforderungen der blockierten Polyisocyante entspricht.

Ein weiterer Gegenstand der vorliegenden Erfindung sind die beanspruchten blockierten Polyisocyanate enthaltenden Klebstoffe, Lacke und Beschichtungssysteme. Die erfindungsgemäßen blockierten Polyisocyanate werden mit den üblichen Harzen für PUR-Beschichtungen, d. h. vorzugsweise mit hydroxylgruppenhaltigen Harzen, kombiniert.

Diese erfindungsgemäßen Verbindungen zeichnen sich dadurch aus, daß sie in Kombination mit einem geeigneten Polyol und in Anwesenheit von geeigneten Katalysatoren bei Einbrennzeiten von 15 bis 30 Minuten bei Temperaturen von 120 bis 140 °C aushärten.

Als Polyole sind z. B. Alkydharze, Polyesterpolymere, hydroxylgruppenhaltige Polyacrylate, hydroxylgruppenhaltige Polyurethanpolymere, hydroxylgruppenhaltige Polyharnstoffpolymere, hydroxylgruppenhaltige Polyetherpolymere, hydroxylgruppenhaltige Epoxypolymere und hydroxylgruppenhaltige Polyepoxid/Amin-Addukte geeignet. Die Epoxypolymere können frei von Epoxidgruppen sein oder Epoxidgruppen enthalten.

Die Molekulargewichte der Polyole können über einen weiten Bereich variieren, je nach Art der Polyole und den gewünschten Eigenschaftsprofilen der aus der Umsetzung mit den erfindungsgemäßen blockierten Polyisocyanaten gebildeten Lacke. Polyester-, Epoxy- und Alkydpolymere können Molekulargewichte im Bereich von 500 bis 50 000 haben, bevorzugt zwischen 1 000 und 5 000. Acrylatpolymere können Molekulargewichte von 100 000 oder höher besitzen. Normalerweise liegen sie im Bereich von 3 000 bis 50 000.

Der Hydroxylgehalt der polymeren Polyole sollte ausreichend groß sein, damit die Zusammensetzung aus Polyol und blockierten Polyisocyanaten zu Lackfilmen mit entsprechenden Gebrauchseigenschaften härtet. Üblicherweise beträgt die Hydroxylzahl des polmeren Polyols mindestens 30, bevorzugt zwischen 70 und 300 mg KOH/g.

Die erfindungsgemäßen blockierten Polyisocyanate stellen wertvolle Vernetzerharze für geeignete Polyole bei der Herstellung von Einbrennlacken, insbesondere bei 1K-PUR-Einbrennlacken dar.

Die Formulierung von Klebstoffen, Lacken und anderen Beschichtungssystemen aus den blockierten Polyisocyanaten und Polyolen ist der Fachwelt bekannt und braucht daher nicht weiter beschrieben zu werden. Das blockierte Polyisocyanat kann eine separate oder eine integrale Komponente zur aktiven Wasserstoff enthaltenden Verbindung sein. So kann z. B. ein Polyisocyanat blockiert sein und als separate Komponente zusammen mit dem Polyol anwesend sein. Alternativ dazu kann das Polyisocyanat auch teilweise blockiert sein und mit einem Polyol zu einer ungelierten Verbindung reagieren. Im letzten Fall bildet das resultierende Urethan einen integralen Bestandteil mit dem Polyol. Das blockierte Polyisocyanat ist in beiden Systemen in ausreichend großer Menge vorhanden, um der härtbaren Zusammensetzung exzellente Härtung zu verleihen. Typischerweise liegt das Äquivalentverhältnis der NCO-Gruppen des Polyisocyanates zum Polyol im Bereich von 0,1:1 bis 2:1.

Von außerordentlicher Bedeutung ist schließlich der Einsatz von Katalysatoren bei der Herstellung hochwertiger Klebstoffe, Lacke und Beschichtungssysteme. Üblicherweise verwendet man bei der Kombination von blockierten Polyisocyanaten mit Harzen Katalysatoren, um eine effektive Härtung bei relativ niedrigen Temperaturen zu ermöglichen. Es werden z. B. Metallsalze und/oder Metallkomplexe in Mengen von ca. 0,1 bis 2,0 Massen-%, besonders 0,5 bis 1,5 Massen-%, bezogen auf das Gewicht der härtbaren Zusammensetzung, eingesetzt. Der Härtungskatalysator kann gleichzeitig mit den anderen Ausgangsmaterialien zur Herstellung der Zusammensetzung gemischt werden oder in die Zusammensetzung in jeder üblichen Reihenfolge zugegeben werden.

Die durch Verwendung der erfindungsgemäßen blockierten Polyisocyanate hergestellten wärmehärtbaren Zusammensetzungen enthalten des weiteren aktiven Wasserstoff tragende Verbindungen und gegebenenfalls Füllstoffe, Pigmente und andere üblicherweise zur Herstellung von wärmehärtbaren Überzügen verwendete Zusatzstoffe.

Der Einsatz der Lackzusammensetzungen gemäß dieser Erfindung gewährleistet eine Härtung des Lackes bei Temperaturen von 120 bis 240 °C, bevorzugt im Temperaturbereich zwischen 120 und 150 °C. So werden hochwertige Beschichtungen oder Lacküberzüge mit geringen Vergilbungswerten erhalten. Bei der Härtung der Lackzusammensetzungen unter den oben angegebenen Bedingungen wurde festgestellt, daß die Systeme nach erfolgtem Einbrennen eine größere Masse auswiesen als erwartet wurde.

Die nachfolgenden Beispiele dienen der näheren Erläuterung der Erfindung.

### Beispiele

### A. Herstellung der erfindungsgemäß als Blockierungsmittel verwendeten α-Hydroxycarbonsäureester

Die Glykolsäure wird im jeweiligen Alkohol gelöst und zu einer vorgelegten Suspension aus Methylisobutylketon und p-Toluolsulfonsäure zugegeben. Das Reaktionsgemisch wird 3-8 h am Rückfluß erhitzt. Aus dem auf Raumtemperatur abgekühlten Gemisch werden unlösliche Bestandteile durch Filtration entfernt. Die anschließende Destillation des Filtrates liefert den α-Hydroxycarbonsäureester. Die Tabelle 1 gibt einen Überblick über die Zusammensetzung und die Darstellung der α-Hydroxycarbonsäureester.

**Tabelle 1**

| α-Hydroxycarbonsäureester | Einsatzstoffe | | | |
|---|---|---|---|---|
| | α-Hydroxycarbonsäure | Einsatzmenge [g] | Alkohol | Einsatzmenge [g] |
| Glykolsäuremethylester | Glykolsäure | 76,05 | Methanol | 96,12 |
| Glykolsäureethylester | Glykolsäure | 76,05 | Ethanol | 116,72 |

### B. Erfindungsgemäße Beispiele 1 bis 4

### Herstellung der mit α-Hydroxycarbonsäureester blockierten Polyisocyanaten

Einem NCO-Äquivalent des bei 40 bis 80 °C in einem inerten Lösemittel gelösten Polyisocyanates werden 0,01 bis 5 Massen-% Dibutylzinndilaurat hinzugefügt. Anschließend werden unter Rühren 0,8 bis 1,2 mol des α-Hydroxycarbonsäureesters portionsweise so zugegeben, daß die Reaktionstemperatur nicht über 100 °C steigt. Nach Beendigung der α-Hydroxycarbonsäureester-Zugabe wird zur Vervollständigung der Reaktion noch 0,5 bis 2 Stunden bei 50 bis 100 °C weiter erhitzt.

Der Tabelle 2 sind die Zusammensetzung, die chemischen und physikalischen Daten der blockierten Isocyanate zu entnehmen.

**Tabelle 2**

| Beispiel Nr. | Blockierte Polyisocyanate | | | | |
|---|---|---|---|---|---|
| | Isocyanat | α-Hydroxycarbonsäureester | freier NCO-Gehalt [Gew.-%] | blockierter NCO-Gehalt [Gew.-%] | Schmelzbereich [° C] |
| 1 | Isocyanat 1 | Glykolsäuremethylester | < 0,1 | 12,4 | 96 - 102 |
| 2 | Isocyanat 2 | Glykolsäureethylester | < 0,1 | 13,4 | - |
| 3 | Isocyanat 1 | Glykolsäurebutylester | < 0,1 | 11,2 | 63 - 65 |
| 4 | Isocyanat 3 | Glykolsäurebutylester | < 0,1 | 12,6 | - |
| Isocyanat 1 = Isocyanurat des 3,5,5-Trimethyl-1-isocyanato-3-isocyanatomethylcyclohexans Isocyanat 2 = Isocyanurat des 3-Methylpentamethylen-1,5-diisocyanats Isocyanat 3 = Isocyanurat des Hexamethylen-1,6-diisocyanats | | | | | |

### C. Erfindungsgemäße Beispiele I bis VI

### Herstellung der erfindungsgemäßen Polyurethan-Lacke

Die in Tabelle 2 aufgeführten blockierten Polyisocyanate werden im stöchiometrischen Verhältnis mit Polyolen gemäß unten beschriebener Rezepturen zu Weißlacken in der üblichen Weise mit TiO₂-Rutil-Typ entsprechend einer PVK 19 pigmentiert und mit geeigneten Additiven formuliert.

Die Polyole sind beispielsweise
- Polyester mit einem Hydroxylgehalt von 1 - 5 Massen-%
- Polyacrylate mit einem Hydroxylgehalt von 1 - 5 Massen-%.

In Tabelle 3 sind die erfindungsgemäßen Beispiele I bis VI Lackzusammensetzungen. Die eingestellte Viskosität beträgt ca. 60 sec, gemessen im DIN-4-Becher bei 20 °C. Alle Zahlenwerte beziehen sich auf Massenprozente.

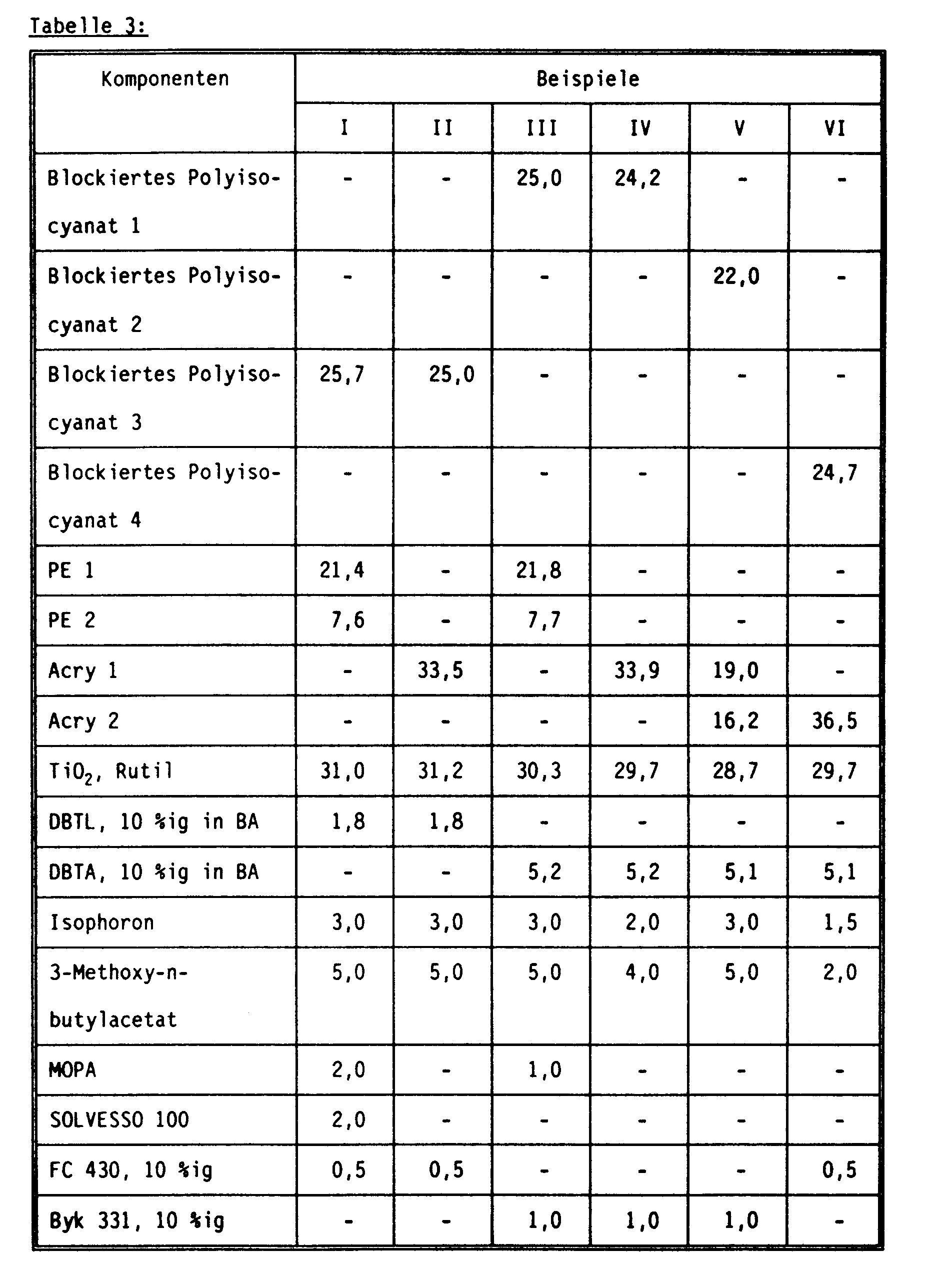

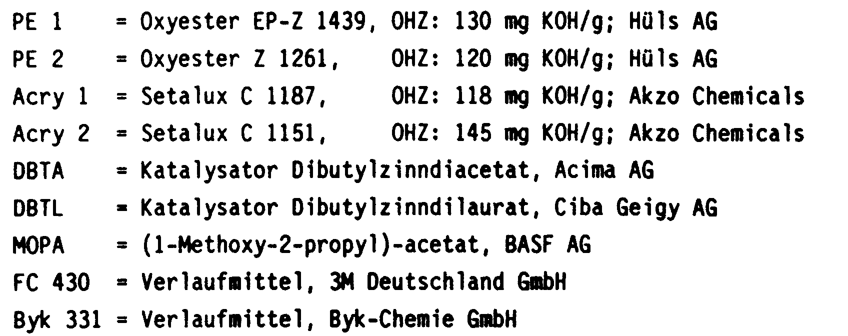

Die formulierten Naßlacke werden mit Hilfe einer Rakel auf 1-mm-Stahlbleche appliziert und unter verschiedenen Härtungsbedingungen in einem Lackumlufttrockenschrank (LUT) gehärtet.

In den folgenden Tabellen 4 und 5 sind wesentliche Verarbeitungsdaten und wichtige lacktechnische Größen zusammengestellt.

**Tabelle 4**

| Beispiel Nr. | Umlufttemperatur im LUT [° C] | | | | | | |
|---|---|---|---|---|---|---|---|
| | 220 | 200 | 180 | 160 | 140 | 130 | 120 |
| | Verweilzeit im LUT [min] | | | | | | |
| I | 2,5 | 3,5 | 6,0 | 12,0 | 30,0 | * | * |
| II | 4,0 | 8,0 | 30,0 | * | * | * | * |
| III | * | * | 4,0 | 5,0 | 15,0 | 30,0 | 45,0 |
| IV | * | * | 3,0 | 4,0 | 8,0 | 20,0 | 45,0 |
| V | * | * | * | 7,0 | 20,0 | 45,0 | * |
| VI | 2,5 | 2,5 | 3,5 | 6,0 | 15,0 | 30,0 | * |

| | | | | | | | |
|---|---|---|---|---|---|---|---|
| *) nicht geprüft | | | | | | | |

Die in Tabelle 5 aufgeführten physikalischen Eigenschaften der Lackfilme sind innerhalb natürlicher Schwankungsbreiten repräsentativ für alle in der Tabelle 4 angegebenen Härtungsbedingungen.

**Tabelle 5**

| Beispiel | SD [µm] | GS | HB | HK [s] | ET [mm] | KS | GG | |
|---|---|---|---|---|---|---|---|---|
| | | | | | | | 20 ° | 60 ° |
| I | 25 - 35 µm | 0 | 125 | 180 | 9 | 60 | 76 | 94 |
| II | 25 - 35 µm | 0 | 125 | 160 | 6 | 50 | 52 | 85 |
| III | 25 - 35 µm | 0 | 118 | 182 | 9 | 60 | 71 | 92 |
| IV | 25 - 35 µm | 0 | 125 | 163 | 9 | 30 | 76 | 91 |
| V | 25 - 35 µm | 0 | 125 | 161 | 8 | 30 | 55 | 90 |
| VI | 25 - 35 µm | 0 | 143 | 168 | 8 | 30 | 49 | 90 |
| SD = Schichtdicke HB = Eindruckhärte Buchholz, nach DIN 53 153 GS = Gitterschnitt, nach DIN 53 151 HK = Härte König, nach DIN 53 157 ET = Erichsentiefung, nach DIN 53 156 KS = Kugelschlag, nach ASTM D-2794/84 GG = Glanz Gardner, nach ASTM D-523 | | | | | | | | |

## Patentansprüche

1. Blockierte Polyisocyanate mit einem freien NCO-Gehalt von kleiner 2 Massen-%,
dadurch gekennzeichnet,
daß sie mit α-Hydroxycarbonsäureestern der allgemeinen Formel I 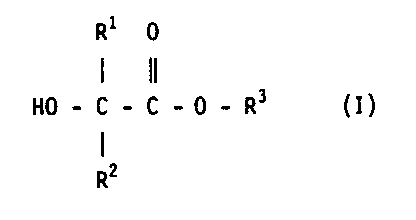 blockiert sind, wobei R¹ und R² gleichzeitig oder unabhängig voneinander Wasserstoff oder Alkyl-, Alkenyl-, Aralkyl- oder Arylgruppen bedeuten und R³ eine Alkyl-, Alkenyl-, Aralkyl- oder Arylgruppe darstellt und wobei diese weitere, auch Heteroatome enthaltende Substituenten aufweisen können.

2. Blockierte Polyisocyanate nach Anspruch 1,
dadurch gekennzeichnet,
daß R¹ gleich Wasserstoff, R² gleich Wasserstoff oder eine Methylgruppe und R³ einen Alkylrest mit 1 bis 10 Kohlenstoffatomen bedeutet.

3. Blockierte Polyisocyanate nach mindestens einem der Ansprüche 1 bis 2,
dadurch gekennzeichnet,
daß aliphatische, cycloaliphatische, aromatische oder heterocyclische Polyisocyanate enthalten sind.

4. Blockierte Polyisocyanate nach mindestens einem der Ansprüche 1 bis 3,
dadurch gekennzeichnet,
daß als Polyisocyanate IPDI, HDI, MPDI, TMDI, H₁₂MDI, MDI, TDI, XDI, TMXDI enthalten sind.

5. Blockierte Polyisocyanate nach mindestens einem der Ansprüche 1 bis 4,
dadurch gekennzeichnet,
daß Polyisocyanate mit Uretdion-, Isocyanurat-, Biuret- oder Allophanatstrukturen enthalten sind.

6. Blockierte Polyisocyanate nach mindestens einem der Ansprüche 1 bis 5,
dadurch gekennzeichnet,
daß sie als Blockierungsmittel Glykolsäuremethylester, Glykolsäureethylester, Glykolsäurebutylester, Milchsäureethylester oder Milchsäurebutylester enthalten.

7. Blockierte Polyisocyanate nach mindestens einem der Ansprüche 1 bis 6,
dadurch gekennzeichnet,
daß sie weitere bekannte Blockierungsmittel enthalten, mit der Maßgabe, daß mindestens 10 Massen-% α-Hydroxycarbonsäureester enthalten sind.

8. Verfahren zur Herstellung von blockierten Polyisocyanaten,
dadurch gekennzeichnet,
daß man Polyisocyanate mit α-Hydroxycarbonsäureestern der allgemeinen Formel I 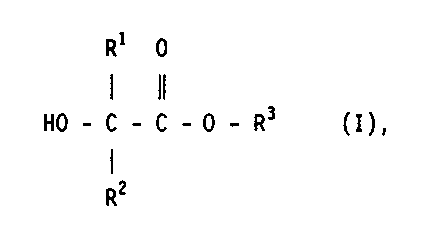 wobei R¹ und R² gleichzeitig oder unabhängig voneinander Wasserstoff oder Alkyl-, Alkenyl-, Aralkyl- oder Arylgruppen bedeuten und R³ eine Alkyl-, Alkenyl-, Aralkyl- oder Arylgruppe darstellt und wobei diese weitere, auch Heteroatome enthaltende Substituenten aufweisen können, so umsetzt, daß pro 0,8 bis 1,2 mol Blockierungsmittel 1 NCO-Äquivalent der Polyisocyanatkomponente in Gegenwart von 0,01 bis 2,0 Massen-% eines Katalysators zur Reaktion kommen.

9. Verfahren nach Anspruch 8,
dadurch gekennzeichnet,
daß R¹ gleich Wasserstoff, R² gleich Wasserstoff oder eine Methylgruppe und R³ einen Alkylrest mit 1 bis 10 Kohlenstoffatomen bedeuten.

10. Lacke und Beschichtungssysteme, im wesentlichen enthaltend mindestens eine aktive wasserstoffhaltige Verbindung und blockierte Polyisocyanate der Ansprüche 1 bis 7.

11. Lacke und Beschichtungssysteme nach Anspruch 10,
dadurch gekennzeichnet,
daß sie Katalysatoren in Mengen von 0,1 bis 2,0 Massen-%, bezogen auf das Gewicht der härtbaren Zusammensetzung, enthalten.

12. 1K-Polyurethan-Einbrennlacke auf Basis von aktiven wasserstoffhaltigen Verbindungen und blockierten Polyisocyanaten der Ansprüche 1 bis 7.

13. Klebstoffe, die die blockierten Polyisocyanate der Ansprüche 1 bis 7 enthalten.
